(19) Europäisches Patentamt
European Patent Office
Office européen des brevets

(11) Veröffentlichungsnummer : **0 104 327 B1**

(12) # EUROPÄISCHE PATENTSCHRIFT

(45) Veröffentlichungstag der Patentschrift : **11.10.89**

(21) Anmeldenummer : **83106267.4**

(22) Anmeldetag : **28.06.83**

(51) Int. Cl.$^4$ : **C 07 C121/00**, C 07 C121/60, C 07 C121/75, C 07 D317/30, C 07 C 13/28, C 07 C 25/18, C 07 C 43/205, C 07 C 69/612, C 09 K 19/20, C 09 K 19/30, C 09 K 19/32

(54) **Verwendung von Ringverbindungen als Komponenten-Flüssigkristallinen-Dielektrika.**

(30) Priorität : **07.07.82 DE 3225290**

(43) Veröffentlichungstag der Anmeldung : **04.04.84 Patentblatt 84/14**

(45) Bekanntmachung des Hinweises auf die Patenterteilung : **11.10.89 Patentblatt 89/41**

(84) Benannte Vertragsstaaten : **CH DE FR GB LI**

(56) Entgegenhaltungen :
EP-A- 0 056 113
EP-A- 0 058 512
EP-A- 0 097 033
DE-A- 3 200 967
DE-A- 3 233 641
FR-A- 2 308 676
GB-A- 2 086 385
GB-A- 2 092 146
GB-A- 2 107 733
US-A- 3 720 690
US-A- 3 787 512
US-A- 3 855 166
Chemical Abstracts Band 93Nr. 8, 25.8.80, s. 888. Ref no. 85211n. Columbus Ohio US
Journal of the Chemical Society, sep/oct 1959.S 2994-2997 MF Ansel et al."Reduced cyclic compounds. Part VIII. The synthesis of (+-)-6-methoxypodocarpa-5,7,13 (14)-trienne &(+-)-abieta-5,7,13 (14)-trienne
Chemical Abstracts Band 33nr. 1 10 Januar 1945, Spalten 292.8-293.9 Columbus Ohio US F Bergmann et al "Substituted stilbenes and 1,4-diphenylbutadienes.II Synthesis and propreties of monohalogeno derivatives"
Molecular Cryst Liq. Band 53, nr. 1/2 1979, S. 147-166 Gordon and Breach Sc. Publ. Ltd. NL. GW Gray "Liquid crystal compounds incorporating the trans 1,4-substituted cyclohexane ring system "

(73) Patentinhaber : **MERCK PATENT GESELLSCHAFT MIT BESCHRÄNKTER HAFTUNG**
**Frankfurter Strasse 250 Postfach 4119**
**D-6100 Darmstadt (DE)**

**The Secretary of State for Defence in Her Britannic Majesty's Government of the United Kingdom of Great Britain and Northern Ireland Whitehall**
**London S.W.1. (GB)**

(72) Erfinder : **Eidenschink, Rudolf, Dr.**
**Erlenweg 17**
**D-6110 Dieburg (DE)**
Erfinder : **Krause, Joachim, Dr.**
**Samuel-Morse-Strasse 14**
**D-6110 Dieburg (DE)**
Erfinder : **Andrews, Beatrice Mary**
**20 Southfield Road**
**Scartho Grimsby South Humberside (GB)**
Erfinder : **Gray, George William**
**33 Newgate Street**
**Cottingham North Humberside HU16 4DY (GB)**
Erfinder : **Carr, Neil**
**19D Pearson Avenue**
**Hull North Humberside HU5 25X (GB)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 104 327 B1

Jouve, 18, rue St-Denis, 75001 Paris, France

**Beschreibung**

Die Erfindung betrifft die Verwendung von Ringverbindungen der Formel I

$$R^1—Q^1—(CH_2)_m—COO_x—(CH_2)_p—Q^2—R^2 \qquad I$$

worin

$R^1$ und $R^2$ jeweils Alkyl oder Alkoxy mit jeweils 1-12 C-Atomen, F, Cl, Br, CN oder $—Q^3—R^3$, einer der Reste $R^1$ und $R^2$ auch H,

$Q^1$, $Q^2$ und $Q^3$ jeweils eine unsubstituierte oder eine durch 1-4 Fluor-, Chlor- und/oder Bromatome substituierte 1,4-Phenylen-, 1,4-Cyclohexylen-, 1,4-Bicyclo(2,2,2) octylen- oder 1,3-Dioxan-2,5-diyl-gruppe,

$R^3$ Alkyl oder Alkoxy mit jeweils 1-8 C-Atomen, H, F, Cl, Br oder CN,

m 1, 2, 3, 4, 5 oder 6, und

p 0, 1, 2, 3, 4 oder 5

bedeuten, wobei die Summe (m + p) 2, 4 oder 6 beträgt als Komponenten flüssigkristalliner Dielektrika.

Der Einfachheit halber bedeuten im folgenden « Phe » eine 1,4-Phenylengruppe, « Cy » eine 1,4-Cyclohexylengruppe, « Bi » eine Bicyclo(2,2,2) octylengruppe und « Dio » eine 1,3-Dioxan-2,5-diylgruppe, wobei diese Gruppen, insbesondere die 1,4-Phenylengruppe, unsubstituiert oder durch 1-4 Fluor-, Chlor- und/oder Bromatome substituiert sein können.

Weiterhin wird der Einfachheit halber die Gruppe $—(CH_2)_m—COO_x—(CH_2)_p—$ im folgenden kurz mit « Z » bezeichnet.

Ähnliche Verbindungen sind z. B. aus der DE-OS 26 17 593 bekannt. Dort ist z. B. eine allgemeine Formel

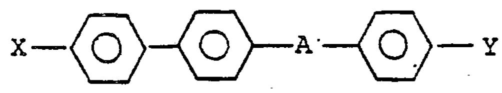

angegeben, worin eine der Gruppen X und Y eine Cyangruppe bedeutet und die andere u.a. auch eine Alkyl- oder Alkylgruppe, die Gruppe A u.a. auch $—(CH_2)_4—$ bedeuten kann.

Die Verbindungen der Formel I können wie ähnliche, z. B. wie die aus der DE-OS 26 17 593 bekannten Verbindungen, als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindungen der Formel I gelöst.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit breitem nematischem Bereich und niedriger Viskosität herstellbar.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die Viskosität oder die Doppelbrechung eines solchen Dielektrikums zu senken.

Die Verbindungen der Formel I sind in reinem Zustand farblos und bilden niedrigviskose flüssigkristalline Mesophasen in einem für die elektrooptische Verwendung günstig gelegenen Temperaturbereich. Chemisch sind sie sehr stabil.

Die Verbindungen der Formel I können nach einem Verfahren hergestellt werden das dadurch gekennzeichnet ist, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt.

oder daß man eine Carbonsäure der Formel II

$$R^1—Q^1—(CH_2)_m—COOH \qquad II$$

worin $R^1$, $Q^1$ und m die angegebene Bedeutung haben oder eines ihrer reaktionsfähigen Derivate mit einem Alkohol bzw. Phenol der Formel III

$$HO-(CH_2)_p-Q^2-R^2 \qquad III$$

worin $R^2$, $Q^2$ und p die angegebene Bedeutung haben
oder mit einem seiner reaktionsfähigen Derivate umsetzt oder daß man zur Herstellung von Dioxanderivaten der Formel I, worin mindestens einer der Reste $Q^1$ bis $Q^3$ eine 1,3-Dioxan-2,5-diylgruppe bedeutet, einen entsprechenden Aldehyd mit einem entsprechenden 1,3-Diol umsetzt
oder daß man zur Herstellung von Nitrilen der Formel I ($R^1$, $R^2$ und/oder $R^3$=CN) ein entsprechendes Carbonsäureamid dehydratisiert
oder daß man zur Herstellung von Ethern der Formel I ($R^1$, $R^2$ und/oder $R^3$ = Alkoxy) eine entsprechende Hydroxyverbindung verethert
und/oder daß man gegebenenfalls eine Chlor- oder Bromverbindung der Formel I ($R^1$, $R^2$ und/oder $R^3$ = Cl oder Br) mit einem Cyanid umsetzt.

Gegenstand der Erfindung ist die Verwendung der Verbindungen der Formel I als Komponenten flüssigkristalliner Dielektrika für elektrooptische Anzeigeelemente. Gegenstand der Erfindung sind weiterhin flüssigkristalline Dielektrika mit einem Gehalt an mindestens einer Verbindung der Formel I sowie electrooptische Anzeigeelemente, die derartige Dielektrika enthalten.

Vor- und nachstehend haben $R^1$, $R^2$, $R^3$, $Q^1$, $Q^2$, $Q^3$, m und p die angegebene Bedeutung, sofern nicht ausdrücklich etwas anderes vermerkt ist.

Die Verbindungen der Formel I umfassen dementsprechend Verbindungen der Teilformeln Ia bis IL :

$R^1-Q^1-CH_2-COO-CH_2-Q^2-R^2$ Ia
$R^1-Q^1-CH_2-COO-(CH_2)_3-Q^2-R^2$ Ib
$R^1-Q^1-CH_2-COO-(CH_2)_5-Q^2-R^2$ Ic
$R^1-Q^1-(CH_2)_2-COO-Q^2-R^2$ Id
$R^1-Q^1-(CH_2)_2-COO-(CH_2)_2-Q^2-R^2$ Ie
$R^1-Q^1-(CH_2)_2-COO-(CH_2)_4-Q^2-R^2$ If
$R^2-Q^1-(CH_2)_3-COO-CH_2-Q^2-R^2$ Ig
$R^1-Q^1-(CH_2)_3-COO-(CH_2)_3-Q^2-R^2$ Ih
$R^1-Q^1-(CH_2)_4-COO-Q^2-R^2$ Ii
$R^1-Q^1-(CH_2)_4-COO-(CH_2)_2-Q^2-R^2$ Ij
$R^1-Q^1-(CH_2)_5-COO-CH_2-Q^2-R^2$ Ik
$R^1-Q^1-(CH_2)_6-COO-Q^2-R^2$ Il

Von diesen sind insbesondere diejenigen der Formel Ia bevorzugt.

Verbindungen der Formel I, die zwei Ringe enthalten, umfassen solche der Teilformeln Im bis Iab, von denen diejenigen der Formeln Im, In, Ig, Ir, Iw and Iab besonders bevorzugt sind :

$R^1$—Phe—Z—Phe—$R^2$ Im
$R^1$—Phe—Z—Cy—$R^2$ In
$R^1$—Phe—Z—Bi—$R^2$ Io
$R^1$—Phe—Z—Dio—$R^2$ Ip
$R^1$—Cy—Z—Phe—$R^2$ Iq
$R^1$—Cy—Z—Cy—$R^2$ Ir
$R^1$—Cy—Z—Bi—$R^2$ Is
$R^1$—Cy—Z—Dio—$R^2$ It
$R^1$—Bi—Z—Phe—$R^2$ Iu
$R^1$—Bi—Z—Cy—$R^2$ Iv
$R^1$—Bi—Z—Bi—$R^2$ Iw
$R^1$—Bi—Z—Dio—$R^2$ Ix
$R^1$—Dio—Z—Phe—$R^2$ Iy
$R^1$—Dio—Z—Cy—$R^2$ Iz
$R^1$—Dio—Z—Bi—$R^2$ Iaa
$R^1$—Dio—Z—Dio—$R^2$ Iab

Von den Verbindungen der Formel I, die drei Ringe enthalten, sind insbesondere diejenigen der Teilformeln Iac bis Iaf bevorzugt :

$R^1$—Phe—Phe—Z—Phe—$R^2$ Iac
$R^1$—Cy—Phe—Z—Phe—$R^2$ Iad
$R^1$—Phe—Z—Phe—Cy—$R^2$ Iae
$R^1$—Cy—Phe—Z—Cy—$R^2$ Iaf

Von den Verbindungen der Formel I, die vier Ringe enthalten, sind insbesondere diejenigen der Teilformeln Iag bis Iam bevorzugt :

$R^1$—Phe—Phe—Z—Phe—Phe—$R^2$ Iag
$R^1$—Cy—Phe—Z—Phe—Cy—$R^2$ Iah
$R^1$—Dio—Phe—Z—Phe—Dio—$R^2$ Iai
$R^1$—Phe—Cy—Z—Cy—Phe—$R^2$ Iaj
$R^1$—Cy—Cy—Z—Cy—Cy—$R^2$ Iak
$R^1$—Dio—Cy—Z—Dy—Dio—$R^2$ Iam

In den Verbindungen der Formel I sowie Ia bis Iam können die Alkyl- bzw. Alkoxyreste geradkettig oder verzweigt sein. Vorzugsweise sind sie geradkettig, haben 2, 3, 4, 5 oder 6 C-Atome und bedeuten demnach bevorzugt Ethyl, Propyl, Butyl, Pentyl, Hexyl, Ethoxy, Propoxy, Butoxy, Pentoxy oder Hexoxy, ferner Methyl, Heptyl, Octyl, Nonyl, Decyl, Undecyl, Dodecyl, Methoxy, Heptoxy, Octoxy, Nonoxy, Decoxy, Undecoxy, Dodecoxy.

Verbindungen der Formeln I sowie Ia bis Iap mit verzweigten Flügelgruppen $R^1$, $R^2$ bzw. $R^3$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Heptylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Im einzelnen bedeuten $R^1$ und $R^2$ bevorzugt Alkyl, Alkoxy oder CN. Unter den Verbindungen der Formeln I sowie Ia bis Iam sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln Ian bis Iat:

$R^1$—Phe—$CH_2$—COO—$CH_2$—$Q^2$—$R^2$ Ian
$R^1$—Phe—$CH_2$—COO—$CH_2$—Phe—$R^3$ Iao
$R^1$—Phe—$CH_2$—COO—$CH_2$—Phe—CN Iap
$R^3$—Phe—$CH_2$—COO—$CH_2$—Phe—$R^3$ Iaq
(worin die beiden Gruppen $R^3$ gleich oder verschieden sein können)
$R^3$—Phe—$CH_2$—COO—$CH_2$—Phe—CN Iar
$R^3$—Cy—Phe—$CH_2$—COO—$CH_2$—Phe—$R^3$ Ias
(worin die beiden Gruppen $R^3$ gleich oder verschieden sein können)
$R^3$—Cy—Phe—$CH_2$—COO—$CH_2$—Phe—CN Iat

In den Verbindungen der Formeln I sowie Ia bis Iat sind diejenigen Stereoisomeren bevorzugt, worin die Substituenten an den Cyclohexylen- und/oder den Dioxandiyl-resten jeweils in trans-Stellung zueinander stehen.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel Ia werden bevorzugt hergestellt, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z. B. freie oder veresterte Hydroxygruppen oder aromatisch gebundene Halogenatome. Bevorzugte Ausgangsstoffe für die Reduktion entsprechen der Formel I, enthalten aber an Stelle der Gruppe $—(CH_2)_m—$ die Gruppe $—CO—(CH_2)_{m-1}—$ und/oder an Stelle der Gruppe $—(CH_2)_p—$ die Gruppe $—(CH_2)_{p-1}—CO—$ und/oder and Stelle eines Cyclohexanrings einen Cyclohexenring.

Ausgangsstoffe mit Cyclohexenringen sind z. B. solche der Formel IV

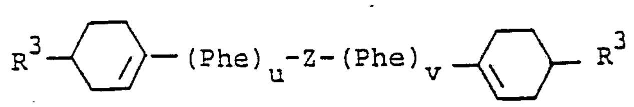

IV

worin u and v jeweils 0 oder 1 bedeuten.

Diese Ausgangsstoffe der Formel IV sind beispielsweise erhältlich durch Umsetzung von 4-$R^3$-Cyclohexanonen mit Organometallverbindungen der Formel Li—$(Phe)_u$—Z—$(Phe)_v$—Li oder entsprechenden Grignardverbindungen und anschließende Dehydratisierung der erhaltenen Carbinole.

Die Reduktion erfolgt zweckmäßig durch katalytische Hydrierung bei Temperaturen zwischen etwa

0° und etwa 200° sowie Drucken zwischen etwa 1 und etwa 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan.

Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Stromtiumcarbonat) oder in feinverteilter Form (z. B. Pt-Mohr) eingesetzt werden können.

Die Ester der Formel I können auch durch Veresterung entsprechender Carbonsäuren der Formel II (oder ihrer reaktionsfähigen Derivate) mit Alkoholen bzw. Phenolen der Formel III (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride der Formel $R^1—Q^1—(CH_2)_m—CO—O—COCH_3$, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metall-alkoholate bzw. Phenolate der Formel $MO—(CH_2)_p—Q^2—R^2$ in Betracht, in der M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie Dimethylformamid (DMF) oder Phosphorsäure-hexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, zum Beispiel Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen —50° und +250°, vorzugsweise zwischen —20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa —25 und +20°.

Dioxanderivate der Formel I (worin eine der Gruppen $Q^1$, $Q^2$ und/oder $Q^3$ eine 1,3-Dioxan-2,5-diylgruppe bedeutet) werden zweckmäßig durch Reaktion eines entsprechenden Aldehyds, z. B. der Formeln $R^1—CHO$, $R^2—CHO$, $O{=}CH—Z—Q^2—R^2$ bzw. $R^1—Q^1—Z—CHO$ (oder eines seiner reaktionsfähigen Derivate) mit einem entsprechenden 1,3-Diol z. B. der Formeln $(HOCH_2)_2CH—Z—Q^2—R^2$, $R^1—Q^1—Z—CH(CH_2OH)_2$, $R^1—CH(CH_2OH)_2$ bzw. $(HOCH_2)_2CH—R^2$ (oder einem seiner reaktionsfähigen Derivate) hergestellt, vorzugsweise in Gegenwart eines inerten Lösungsmittels wie Benzol oder Toluol und/oder eines Katalysators, z. B. einer starken Säure wie Schwefelsäure, Benzol- oder p-Toluolsulfonsäure, bei Temperaturen zwischen etwa 20 und etwa 150, vorzugsweise zwischen 80 und 120°. Als reaktionsfähige Derivate der Ausgangsstoffe eignen sich in erster Linie Acetale z. B. der Formeln $R^1—CH(OR^4)_2$, $R^2—CH(OR^4)_2$, $(R^4O)_2CH—Z—Q^2—R^2$, $R^1—Q^1—Z—CH(OR^4)_2$, $R^5—CH(OCH_2)_2CH—Z—Q^2—R^2$, $R^1—Q^1—Z—CH(CH_2O)_2CH—R^5$, $R^1—CH(CH_2O)_2—CH—R^5$ bzw. $R^5—CH(OCH_2)_2CH—R^2$, worin $R^4$ Alkyl mit 1-4 C-Atomen, zwei Reste $R^4$ zusammen auch Alkylen mit 2 oder 3 C-Atomen und $R^5$ H, Alkyl mit 1-4 C-Atomen oder Phenyl bedeuten.

Die genannten Aldehyde und 1,3-Diole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester erhältlich.

Zur Herstellung von Nitrilen der Formel I ($R^1$, $R^2$ und/oder $R^3$ = CN), insbesondere solchen der Formeln Iap, Iar und Iat, können entsprechende Säureamide, z. B. solche der Formeln

$H_2NCO—Q^1—Z—Q^2—R^2$ oder $R^1—Q^1—Z—Q^2—CONH_2$ dehydratisiert werden. Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z. B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0 und 150° arbeiten ; als Lösungsmittel kommen z. B. Basen wie Pyridin oder Triethylamin, aromatische Kohlenwasserstoffe wie Benzol, Toluol oder Xylol oder Amide wie DMF in Betracht.

Alkoxyverbindungen der Formel I ($R^1$, $R^2$ und/oder $R^3$ = Alkoxy) sind durch Alkylierung entsprechender Hydroxyverbindungen, vorzugsweise entsprechender Phenole, erhältlich, wobei die Hydroxyverbindung zweckmäßig zunächst in ein entsprechendes Metallderivat, z. B. durch Behandeln mit NaOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, DMF oder Dimethylsulfoxid oder auch einem Überschuß an wässeriger oder wässerigalkoholischer NaOH oder KOH bei Temperaturen zwischen etwa 20 und 100°.

Zur Herstellung von Nitrilen der Formel I ($R^1$, $R^2$ und/oder $R^3$ = CN) können auch entsprechende Chloroder Bromverbindungen der Formel I ($R^1$, $R^2$ und/oder $R^3$ = Cl oder Br) mit einem Cyanid umgesetzt werden, zweckmäßig mit einem Metallcyanid wie NaCN, KCN oder $Cu_2(CN)_2$, z. B. in Gegenwart von Pyridin in einem inerten Lösungsmittel wie DMF oder N-Methylpyrrolidon bei Temperaturen zwischen 20 und 200°.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel charakterisieren,

$$R^6—A—G—E—R^7 \qquad V$$

worin A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | |
|---|---|
| —CH=CH— | —N(O)=N— |
| —CH=CY— | —CH=N(O)— |
| —C≡C— | $—CH_2—CH_2—$ |
| —CO—O— | $—CH_2—O—$ |
| —CO—S— | $—CH_2—S—$ |
| —CH=N— | —COO—Phe—COO— |

oder eine C-C-Einfachbindung, Y Halogen, vorzugsweise Chlor, oder —CN, und $R^6$ und $R^7$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind $R^6$ und $R^7$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrika enthalten in allgemeinen 0,1 bis 60, vorzugsweise 5 bis 40 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vergl. z. B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind zum Beispiel in den DE-OS 22 09 127, 22 40 864, 23 21 632, 23 38 281, 24 50 088, 26 37 430, 28 53 728 und 29 02 177 beschrieben.

Die folgenden Beispiele sollen die Herstellung der Verbindungen der Formel I erläutern, F. = Schmelzpunkt, K. = Klärpunkt. Vor- und nachstehend bedeuten Prozentangaben Gewichtsprozent ; alle Temperaturen sind in Grad Celsius angegeben.

Beispiel 1

Man kocht 28,8 g p-(trans-4-Pentylcyclohexyl)-phenyl-essigsäure 1 Std. mit 24 g $SOCl_2$, dampft ein, löst das erhaltene rohe Säurechlorid in 150 ml Toluol, versetzt mit 8 ml Pyridin und 13,3 g p-Cyanbenzylalkohol und kocht 2 Stunden. Nach Abkühlen und üblicher Aufarbeitung erhält man p-(trans-4-Pentylcyclohexyl)-phenyl-essigsäure-(p-cyanbenzylester), F. 85°, K. 30°.

Analog erhält man durch Veresterung der entsprechenden Säuren :

p-Methoxyphenylessigsäure-(p-cyanbenzylester)
p-Ethoxyphenylessigsäure-(p-cyanbenzylester)
p-Propoxyphenylessigsäure-(p-cyanbenzylester)
p-(trans-4-Propylcyclohexyl)-phenylessigsäure-(p-cyanbenzylester)
p-(trans-4-Butylcyclohexyl)-phenylessigsäure-(p-cyanbenzylester)
p-(trans-4-Pentylcyclohexyl)-phenylessigsäure-(p-fluorbenzylester)
p-(trans-4-Pentylcyclohexyl)-phenylessigsäure-(p-chlorbenzylester)
p-(trans-4-Pentylcyclohexyl)-phenylessigsäure-(p-brombenzylester)
p-Methoxyphenylessigsäure-(trans-4-propylcyclohexylmethylester)
p-Cyanphenylessigsäure-(3-p-cyanphenylpropylester)
p-Fluorphenylessigsäure-(5-p-cyanphenylpentylester)
3-Phenylpropionsäure-(p-cyanphenylester)
3-(p-trans-4-Propylcyclohexyl-phenyl)-propionsäure-(p-cyanphenylester), F. 91°, K. 108°.
3-(p-trans-4-Propylcyclohexyl-phenyl)-propionsäure-(p-ethoxyphenylester)
3-(p-trans-4-Propylcyclohexyl-phenyl)-propionsäure-(p-butoxyphenylester), F. 75°, K. 101°
3-(p-trans-4-Propylcyclohexyl-phenyl)-propionsäure-(p-propylphenylester)
3-(p-trans-4-Propylcyclohexyl-phenyl)-propionsäure-(p-pentylphenylester), F. 65°, K. 74°
3-(p-trans-4-Propylcyclohexyl-phenyl)-propionsäure-(p-heptylphenylester)
3-(p-trans-4-Propylcyclohexyl-phenyl)-propionsäure-(p-fluorphenylester), F. 97°, K 55° (monotrop)
3-(p-trans-4-Pentylcyclohexyl-phenyl)-propionsäure-(p-cyanphenylester)
3-(p-trans-4-Pentylcyclohexyl-phenyl)-propionsäure-(p-ethoxyphenylester)
3-(p-trans-4-Pentylcyclohexyl-phenyl)-propionsäure-(p-butoxyphenylester)
3-(p-trans-4-Pentylcyclohexyl-phenyl)-propionsäure-(p-propylphenylester)
3-(p-trans-4-Pentylcyclohexyl-phenyl)-propionsäure-(p-pentylphenylester)
3-(p-trans-4-Pentylcyclohexyl-phenyl)-propionsäure-(p-heptylphenylester)
3-(p-trans-4-Pentylcyclohexyl-phenyl)-propionsäure-(p-fluorphenylester)
3-p-Methoxyphenylpropionsäure-(2-p-cyanphenylethylester)
3-p-Chlorphenylpropionsäure-(4-p-cyanphenylbutylester)
4-p-Ethoxyphenylbuttersäure-(p-cyanbenzylester)
4-p-Ethoxyphenylbuttersäure-(3-p-cyanphenylpropylester)
5-p-Ethoxyphenylpentansäure-(p-cyanphenylester)
5-p-Ethoxyphenylpentansäure-(2-p-cyanphenylethylester)
6-p-Ethoxyphenylhexansäure-(p-cyanbenzylester)
7-p-Ethoxyphenylheptansäure-(p-cyanphenylester).

Beispiel 2

Eine Lösung von 31,3 g p-Ethoxyphenylessigsäure-(p-carbamoylbenzylester (erhältlich durch Veresterung) in 500 ml DMF wird bei 50° unter Rühren tropfenweise mit 65 g $POCl_3$ versetzt. Nach weiterem einstündigem Rühren gießt man auf Eis, arbeitet wie üblich auf und erhält p-Ethoxyphenylessigsäure-(p-cyanbenzylester), F. 59°, K. — 40°.

Beispiel 3

Ein Gemisch aus 34,9 g p-Ethoxyphenylessigsäure-(p-brombenzylester) (erhältlich durch Veresterung), 10 g $Cu_2(CN_2)$, 120 ml Pyridin und 60 ml N-Methylpyrrolidon wird 2 Std. auf 150° erhitzt. Man kühlt ab, gibt eine Lösung von 120 g $FeCl_3 \cdot 6H_2O$ in 600 ml 20 %iger Salzsäure hinzu, erwärmt 1,5 Std. unter Rühren auf 70°, arbeitet wie üblich auf un erhält p-Ethoxyphenylessigsäure-(p-cyanbenzylester), F. 59°, K. — 40°.

## Patentansprüche

1. Verwendung von Ringverbindungen der Formel I

$$R^1—Q^1—(CH_2)_m—COO—(CH_2)_p—Q^2—R^2 \qquad I$$

worin

$R^1$ und $R^2$ jeweils Alkyl oder Alkoxy mit jeweils 1-12 C-Atomen, F, Cl, Br, CN oder $—Q^3—R^3$, einer der Reste $R^1$ und $R^2$ auch H,

$Q^1$, $Q^2$ und $Q^3$ jeweils eine unsubstituierte oder eine durch 1-4 Fluor-, Chlor- und/oder Bromatome substituierte 1,4-Phenylen-, 1,4-Cyclohexylen-, 1,4-Bicyclo(2,2,2) octylen- oder 1,3-Dioxan-2,5-diyl-gruppe,

$R^3$ Alkyl oder Alkoxy mit jeweils 1-8 C-Atomen, H, F, Cl, Br oder CN,

m 1, 2, 3, 4, 5 oder 6 und

p 0, 1, 2, 3, 4 oder 5

bedeuten, wobei die Summe (m + p) 2, 4 oder 6 beträgt als Komponenten flüssigkristalliner Dielektrika für elektrooptische Anzeigeelemente.

2. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

3. Elektrooptisches Anzeigeelement, dadurch gekennzeichnet, daß es ein Dielektrikum nach Anspruch 2 enthält.

## Claims

1. Use of ring compounds of the formula I

$$R^1—Q^1—(CH_2)_m—COO—(CH_2)_p—Q^2—R_2 \qquad I$$

wherein

$R^1$ and $R^2$ are each alkyl or alkoxy each of which has 1-12 C atoms, F, Cl, Br, CN or $—Q^3—R^3$, one of the radicals $R^1$ and $R^2$ is also H,

$Q^1$, $Q^2$ and $Q^3$ are each a 1,4-phenylene, 1,4-cyclohexylene, 1,4-bicyclo(2,2,2)-octylene or 1,3-dioxane-2,5-diyl group which is unsubstituted or substituted by 1-4 fluorine, chlorine and/or bromine atoms,

$R^3$ is alkyl or alkoxy each of which has 1-8 C atoms, H, F, Cl, Br or CN,

m is 1, 2, 3, 4, 5 or 6, and

p is 0, 1, 2, 3, 4 or 5,

the sum of (m + p) being 2, 4 or 6, as components of liquid-crystal dielectrics for electro-optical display elements.

2. Liquid-crystal dielectric for electro-optical display elements containing at least two liquid-crystal components, characterised in that at least one of the components is a compound of the formula I according to Claim 1.

3. Electro-optical display element characterised in that it contains a dielectric according to Claim 4.

## Revendications

1. Utilisation des dérivés cycliques de formule I

$$R^1—Q^1—(CH_2)_m—COO—(CH_2)_p—Q^2—R^2 \qquad I$$

où

$R^1$ et $R^2$ représentent un alkyle ou un alcoxy ayant 1 à 12 atomes de C, représentent F, Cl, Br, CN ou $—Q^3—R^3$, l'un des restes $R^1$ et $R^2$ représente également H,

$Q^1$, $Q^2$ et $Q^3$ représentent un groupe 1,4-phénylène, un groupe 1,4-cyclohexylène, un groupe 1,4-bicyclo(2,2,2) octylène ou un groupe 1,3-dioxan-2,5-diyle non substitués ou substitués par 1 à 4 atomes de fluor, de chlore et/ou de brome,

$R^3$ représente un alkyle ou un alcoxy ayant 1 à 8 atomes de C, représente H, F, Cl, Br ou CN,

m est 1, 2, 3, 4, 5 ou 6 et

p est 0, 1, 2, 3, 4 ou 5,

la somme (m + p) étant 2, 4 ou 6 comme composant diélectrique à cristaux liquides pour élément d'affichage électro optique.

2. Diélectrique à cristaux liquides pour éléments d'affichage avec au moins deux composants à cristaux liquides, caractérisé en ce qu'au moins un composant est un composé de formule (I) selon la revendication 1.

3. Elément d'affichage électro-optique, caractérisé en ce qu'il contient un diélectrique selon la revendication 2.